# EUROPÄISCHE PATENTSCHRIFT

(11) **EP 1 674 127 B1**
(45) Veröffentlichungstag und Bekanntmachung des Hinweises auf die Patenterteilung: **20.08.2008**
(21) Anmeldenummer: 05027818.3
(22) Anmeldetag: 19.12.2005
(51) Int. Cl.: A61M 27/00

(54) **Vorrichtung zum Entfernen von Wundsekreten**
Device to remove wound secretion
Instrument permettant d' enlever les sécrétions d'une plaie

(30) Priorität: 21.12.2004 DE 102004062877
(43) Veröffentlichungstag der Anmeldung: 28.06.2006
(73) Patentinhaber: Ihle, Peter, 37327 Birkungen (DE); Weisse, Steffen, Dr., 99755 Ellrich (DE)
(72) Erfinder: Ihle, Peter, 37327 Birkungen (DE)
(74) Vertreter: Carlsohn, Alexander

(56) Entgegenhaltungen:
- EP-A- 0 265 906
- WO-A-01/85228
- WO-A-94/20041
- US-A- 5 298 015
- US-A1- 2002 150 720
- US-B1- 6 458 109
- US-B1- 6 695 823

## Beschreibung

Die Erfindung betrifft eine Vorrichtung zum Entfernen von Wundsekreten aus Körperhöhlen. Sie betrifft ferner ein Kit, das zum Entfernen von Wundsekreten aus Körperhöhlen geeignet ist. Die Vorrichtung und das Kit sind insbesondere zum permanenten Entfernen von Wundsekreten aus Körperhöhlen geeignet.

Wundsekrete sind überwiegend wässerige Absonderungen, die aufgrund einer Verletzung aus einer Wunde austreten können. Die Zusammensetzung der Wundsekrete, die vor allem aus den Zellzwischenräumen gespeist werden, kann stark variieren. Es enthält vorwiegend Beimengungen von Serumproteinen und Spuren von Blutinhaltsstoffen. Bei einer Infektion enthalten Wundsekrete überdies erhöhte Mengen an Makrophagen, die die Mikroorganismen beseitigen sollen. Dies kann mit der Entstehung von Eiter verbunden sein. Wird das Wundsekret nicht aus einer entzündeten Körperhöhle entfernt, führt dies zu Bildung von Abszessen.

Zum Entfernen von Wundsekreten aus Körperhöhlen werden in der Praxis verschiedene Vorrichtungen verwendet. Bekannt ist die Verwendung von Drainageschläuchen, Bauchtüchern oder kleineren Schwämmen.

Die bekannten Vorrichtungen sind jedoch mit Nachteilen verbunden. Der Einsatz von Drainageschläuchen führt sehr schnell zur Bildung von Kanälen in der Körperhöhle. Das in der Körperhöhle befindliche und entstehende Wundsekret wird somit nur dann abgesaugt, wenn es sich in der Nähe eines solchen Kanals befindet. Im übrigen verbleibt es aber in der Körperresthöhle. Drainageschläuche sind daher zum permanenten und möglichst vollständigen Absaugen von Wundsekreten nicht geeignet. Drainageschläuche bestehen zumeist aus Silikon.

Bauchtücher sind in der Regel rechteckige Lagen aus Baumwolle oder einem vergleichbaren weichen und saugfähigen Material, die in die Körperhöhle eingebracht werden. Das saugfähige Material nimmt das in der Körperhöhle befindliche oder entstehende Wundsekret auf. Naturgemäß hat ein solches Bauchtuch jedoch nur eine begrenzte Kapazität zur Aufnahme von Körperflüssigkeiten. Aus diesem Grund ist es erforderlich, das Bauchtuch nach einer gewissen Zeit aus der Körperhöhle zu entfernen und durch ein neues zu ersetzen. Dieser Austausch der Bauchtücher ist jeweils mit einem operativen Eingriff und einer permanenten Narkose verbunden, der den physischen und psychischen Zustand des Patienten nachteilig beeinflussen kann. Überdies hat der Behandler, der ein Bauchtuch in eine Körperhöhle einbringt, dafür Sorge zu tragen, daß dieses ersetzt wird, bevor dessen Kapazität tatsächlich erschöpft ist. Aus diesem Grunde hat der Behandler Mußmaßungen darüber anzustellen, wann dies der Fall sein wird.

Schwämme sind zu derb und unflexibel, um sie in Körperhöhlen einbringen zu können. Da die Schwämme starr und zu klein sind, können sie lediglich in kleinen Körperhöhlen eingesetzt werden. Aufgrund der Beschaffenheit der Schwämme ist mit einer Schädigung von Organoberflächen zu rechnen. Die nicht besaugten Abschnitte der Körperhöhle verkleben und können sich entzünden. Sie sind daher zum Absaugen von Wundsekreten aus Körperhöhlen nicht oder nur äußerst begrenzt geeignet. Schwämme werden daher vorwiegend zur Behandlung von äußeren Wunden eingesetzt. Dazu wird der Schwamm auf die Wunde aufgebracht, die Wunde und der darauf befindliche Schwamm mit einer Vakuumfolie abgedeckt und der Schwamm mit einem Drainageschlauch verbunden, über den ein Unterdruck zwischen der Oberseite der äußeren Wunde und der Unterseite der Vakuumfolie erzeugt werden kann. Das Wundsekret kann so aus der offenen Wunde abgesaugt werden. Der Schwamm kann durch Zuschneiden an die Wundgröße angepaßt werden. Der Schwamm muß jedoch nicht nur die Wunde bedecken, sondern auch eine bestimmte Dicke über der Wunde aufweisen, um für eine gleichmäßige Verteilung des Unterdruckes über die Wundoberfläche zu sorgen. Besonders nachteilig ist jedoch die Gefahr, daß der Schwamm verstopft, so daß der Unterdruck nicht mehr gleichmäßig über die Wundoberfläche verteilt werden kann.

US 2002/150720 A1 offenbart einen Wundverband Abdeckung äußerer Wunden, umfassend eine erste Schicht aus einem Material, das in Kontakt mit der Wunde gebracht werden kann und als Wundenkontaktschicht dient. Dasselbe gilt jedoch nicht für die zweite Schicht. Ferner ist eine zweite Schicht vorgesehen, die zum Schutz der darunterliegenden Strukturen des Wundverbandes vor Kontaminationen und vor einem Flüssigkeitsverlust dient. Überdies soll die zweite Schicht eine Rückschicht aufweisen, wobei die Rückschicht bevorzugt eine Feuchtigkeitssperre bereitstellt.

US-B1-6 458 109 offenbart eine erste Bandage zur Abdeckung von Wunden. Die zweite Bandage dient zur Abdeckung der ersten Bandage.

EP-A-0 256 906 zeigt einen Wundverband, der aus einem ersten Teil mit Kontakt zu einer Wunde und einem zweiten Teil, der von der Wundoberfläche entfernt ist, besteht.

Aus dem Stand der Technik ist somit kein System bekannt, daß eine permanente und vollständige Entfernung von Wundsekreten aus Körperhöhlen ermöglicht, ohne daß ständige operative Eingriffe erforderlich sind. Es gibt insbesondere kein System, mit dem die gesamte Bauchhöhle einschließlich aller Organzwischenräume abgesaugt werden kann, ohne die Organe und deren Oberfläche zu schädigen.

Aufgabe der Erfindung ist es, die Nachteile nach dem Stand der Technik zu beseitigen. Es soll insbesondere ein Vorrichtung angegeben werden, die einerseits eine permanente und vollständige Entfernung von Wundsekreten aus der gesamten Körperhöhle und andererseits eine einfache Anpassung der Vorrichtung an die Größe der Körperhöhle ermöglicht. Ferner sollen ein Kit zur Herstellung einer solchen Vorrichtung und ein Verfahren zur Herstellung der Vorrichtung angegeben werden.

Diese Aufgabe wird durch die Merkmale der Ansprüche 1 und 12 gelöst. Zweckmäßige Ausgestaltungen der Erfindungen ergeben sich aus den Merkmalen der Ansprüche 2 bis 11 und 13.

Nach Maßgabe der Erfindung ist eine Vorrichtung zum Entfernen von Wundsekreten aus Körperhöhlen vorgesehen, umfassend
(a) zumindest zwei aufeinanderliegende Lagen eines Schaumstoffkörpers aus offenporigem Polyurethan, wobei das Polyurethan eine Porengröße von 10 bis 75 ppi und eine Rohdichte von 14 bis 75 kg/m³ aufweist, und
(b) zumindest einen Schlauch mit offenen Enden, dessen eines offene Ende zwischen zwei benachbarte Lagen des Schaumstoffkörpers eingebracht ist, wobei sich das zweite offene Ende des Schlauches außerhalb der Lagen befindet;
dadurch gekennzeichnet, daß die Lagen keine Beschichtung aufweisen und daß, nach dem Einbringen der Vorrichtung in die Körperhöhle, die Außenseiten der äußeren Lagen an den Innenseiten der Körperhöhle anliegen, so daß das Sekret über die Poren der beiden äußeren Lagen in den Schaumstoffkörper eintreten kann.

Das zweite offene Ende, daß sich außerhalb der Lagen befindet, wird zweckmäßigerweise an eine Saugpumpe angeschlossen, so daß ein Unterdruck in der Körperhöhle erzeugt werden kann.

Die Vorrichtung ist sterilisierbar und steril anzuwenden.

Die Lagen des Schaumstoffkörpers werden in eine Körperhöhle eingebracht. Die Lagen sind offenporig und weisen keine Beschichtung, beispielsweise in Form einer Folie, auf, die die Poren der Lagen verschließt. Werden die Lagen in die Körperhöhle eingebracht, so liegen die Außenseiten der beiden äußeren Lagen jeweils an den Innenseiten der Körperhöhle an, so daß sich dort bildendes Wundsekret über die Poren der Lagen in diese eintreten kann. Die Lagen liegen aufeinander, sind aber nicht miteinander verbunden. Vielmehr ist der Unterdruck in einer Körperhöhle wie der Peritonealhöhle ausreichend, um die Lagen aufeinander zu pressen.

Unter einer Körperhöhle wird hier ein Hohlraum des menschlichen oder tierischen Körpers verstanden. Solche Hohlräume sind in der Regel mit einem Epithel oder Mesothel ausgekleidet. Die erfindungsgemäße Vorrichtung kann beispielsweise zum Entfernen von Wundsekreten aus der Peritonealhöhle (Cavitas peritonealis), Pleurahöhle (Cavitas pleuralis) oder Gelenkhöhlen (Cavitas articularis) verwendet werden.

Die erfindungsgemäße Vorrichtung ermöglicht es erstmals, permanent Wundsekret aus einer Körperöffnung abzusaugen. Im Gegensatz zu den Drainageschläuchen des Standes der Technik wird die Kanalbildung durch das Einbringen der Lagen verhindert. Die Lagen des Schaumstoffmaterials sind im Vergleich zum den Schwämmen dünn und flexibel und ermöglichen aufgrund der Porengestaltung längerfristiges, kontinuierliches und organschonendes Absaugen von Wundsekreten aus Körperhöhlen. Die Gefahr einer Verletzung von Organen ist weitestgehend ausgeschlossen.

Eine Lage eines Schaumstoffkörpers sollte eine Stärke von 1 bis 50 mm, vorzugsweise eine Stärke von 3 bis 15 mm aufweisen. In einer besonders bevorzugten Ausführungsform liegt die Stärke einer Lage zwischen dem 0,2- bis 0,8fachen, stärker bevorzugt dem 0,4- bis 0,6fachen des Schlauchaußendurchmessers. Besonders bevorzugt ist es, wenn die Stärke einer Lage dem 0,5fachen des Schlauchaußendurchmessers entspricht.

Das offenporige Polyurethan weist erfindungsgemäß eine Porengröße von 10 bis 75 ppi auf. Dies entspricht 4 bis 25 Poren pro Zentimeter Oberfläche des Schaumstoffkörpers. Bevorzugt hat der offenporige Polyurethan 5 bis 20 Poren pro Zentimeter Oberfläche des Schaumstoffkörpers.

Der offenporige Polyurethan hat erfindungsgemäß eine Rohdichte von 14 bis 75 kg/m³, bevorzugt eine Rohdichte von 20 bis 40 kg/m³. Unter Rohdichte wird hier die Dichte des Polyurethans vor dem Eindringen von Wundsekret verstanden.

Die Lagen aus dem offenporige Polyurethan können im Gegensatz zum Stand der Technik verhältnismäßig einfach an die mit der Vorrichtung auszukleidende Körperhöhle angepaßt werden. Die Höhe der Vorrichtung wird durch die Anzahl der aufeinanderliegenden Lagen bestimmt. Die Länge und Breite der Lagen kann durch Zuschneiden bestimmt werden.

Der Schlauch besteht vorzugsweise aus Silikon oder einem anderen weichen, flexiblen und physiologisch akzeptablen Kunststoff. Der Außendurchmesser des Schlauches ergibt sich vorzugsweise aus der Stärke einer Lage des Schaumstoffmaterials.

Vorzugsweise ist das offene Ende des Schlauches, das sich zwischen den benachbarten Lagen befindet, so zwischen den Lagen angeordnet, daß sich daß offene Ende nicht am Rand der Lagen befindet. Vielmehr sollte ein Teil des Schlauches zwischen den beiden Lagen verlaufen. Vorzugsweise beträgt die Länge des Teil des Schlauches, das zwischen den beiden benachbarten Lagen verläuft, zumindest 50 %, stärker bevorzugt 60 % der Länge der Lagen.

Zweckmäßigerweise weist der Teil des Schlauches, der sich zwischen den beiden Lagen des Schaumstoffkörpers befindet, neben dem offenen Ende weitere Öffnungen auf, die im Mantel des Schlauches ausgebildet sind. Zweckmäßigerweise sind die weiteren Öffnungen gleichmäßig über den Teil des Schlauches, das sich zwischen den beiden Lagen befindet, verteilt. Die Öffnungen können in Abhängigkeit von dem Schlauchaußendurchmesser 1 bis 15 mm groß sein. Bevorzugt gleicht die Größe jeder Öffnung der Größe des offenen Endes des Teiles des Schlauches, der sich zwischen den beiden Lagen befindet. Alternativ kann vorgesehen sein, daß die Größe der weiteren Öffnungen mit zunehmender Entfernung von dem offenen Ende des Teils des Schlauches, das sich zwischen den beiden Lagen befindet, zunehmen kann. In diesem Fall sollte die größte weitere Öffnung so groß wie das offene Ende des Schlauches sein.

Die weiteren Öffnungen sind vorzugsweise runde oder elliptische Öffnungen; sie können aber auch jede andere geeignete Form aufweisen.

Der Abstand zwischen zwei benachbarten Öffnungen sowie der Öffnung, die dem offenen Ende des Teiles des Schlauches, der sich zwischen den beiden Lagen befindet, benachbart ist, sollte zwischen dem Zweifachen und dem Zehnfachen des Durchmessers der größten Öffnung liegen.

In einer bevorzugten Ausführungsform sind die Öffnungen auf dem Schlauchmantel versetzt zueinander angeordnet. Die Öffnungen sind vorzugsweise auf einer gedachten Spirale über dem Schlauchmantel verteilt.

Der Teil des Schlauches, der sich zwischen den beiden Lagen befindet, sollte so zwischen die Lagen eingebracht werden, daß die obere Außenfläche des Schlauchmantels in Kontakt mit der Unterseite der oberen Lage und die untere Außenfläche des Schlauchmantels in Kontakt mit der Oberseite der unteren Lage steht, und zwar über den gesamten Teil des Schlauches hin, der sich zwischen den beiden Lagen befindet. Der zwischen den Lagen befindliche Teil des Schlauches kann zwischen den Lagen gerade oder gekrümmt verlaufen. Er kann beispielsweise S-förmig gekrümmt sein. Ist der Schlauch gerade, so sollte er mittig zwischen den Rändern der Lagen angeordnet sein.

Überdies können mehrere Schläuche zwischen zwei benachbarte Lagen eingebracht werden. Beispielsweise kann zwischen zwei benachbarte Lagen ein zweiter Schlauch mit offenen Enden eingebracht sein, dessen eines offene Ende zwischen die beiden Lagen des Schaumstoffkörpers eingebracht ist, wobei sich das zweite offene Ende des zweiten Schlauches außerhalb der Lagen befindet. Der Abstand des Teils des zweiten Schlauches, das sich zwischen den beiden Lagen befindet, von dem Teil des ersten Schlauches, entspricht in einer bevorzugten Ausführungsform der Hälfte der Breite einer Lage des Schaumstoffmaterials, wobei die Enden dieser Teile der Schläuche parallel zueinander angeordnet sind.

Das Ende eines jeden Schlauches, das sich außerhalb der Lagen befindet, sollte mit einer Einrichtung zur Erzeugung von Unterdruck verbunden sein. Die Saugpumpe sollte einen Unterdruck zwischen 10 und 250 mm Hg erzeugen. Auf diese Weise wird in der Körperhöhle ein Unterdruck erzeugt, so daß in der Körperhöhle befindendes oder entstehendes Wundsekret in den Schaumstoffkörper eintritt, von dort über dessen Poren zu den Öffnungen des Schlauches gelangt und schließlich über den Schlauch nach außen aus der Körperhohle heraus gesaugt wird. Die Saugpumpe kann jede Pumpe sein, die einen Unterdruck erzeugen kann, beispielsweise eine maschinelle Pumpe oder eine Handpumpe.

Die erfindungsgemäße Vorrichtung kann mehr als zwei Lagen umfassen. Zwischen benachbarten Lagen sollte jeweils zumindest ein Schlauch eingebracht werden, dies ist aber nicht erforderlich, sofern die Vorrichtung insgesamt zumindest einen Schlauch umfaßt. Wenn mehr als drei Lagen vorgesehen sind, wird vorzugsweise jedoch zwischen die beiden obersten Lagen zumindest ein Schlauch und zwischen die beiden untersten Lagen zumindest ein Schlauch eingebracht. Unabhängig davon können zwischen benachbarte Lagen jeweils ein, zwei oder mehr Schläuche eingebracht werden.

In einer bevorzugten Ausführungsform besteht die Vorrichtung aus den Lagen und dem zumindest einen Schlauch.

Nach Maßgabe der Erfindung ist ferner ein Kit zur Herstellung einer Vorrichtung zum Entfernen von Wundsekreten aus Körperöffnungen vorgesehen. Dieses Kit umfaßt
(a) eine oder mehrere Lagen eines Schaumstoffkörpers aus offenporigem Polyurethan, wobei das Polyurethan eine Porengröße von 10 bis 75 ppi und eine Rohdichte von 14 bis 75 kg/m³ aufweist, und
(b) einen oder mehrere Schläuche mit offenen Enden, dessen eines offene Ende zwischen die beiden Lagen des Schaumstoffkörpers eingebracht wird, wobei sich das zweite offene Ende des Schlauches außerhalb der Lagen befindet;
dadurch gekennzeichnet, daß die Lagen keine Beschichtung aufweisen und daß, nach dem Einbringen der Vorrichtung in die Körperhöhle, die Außenseiten der äußeren Lagen an den Innenseiten der Körperhöhle anliegen, so daß das Sekret über die Poren der beiden äußeren Lagen in den Schaumstoffkörper eintreten kann.

Die Lagen können in Form einer flexiblen Binde, Rolle oder Platte bereitgestellt werden. Sie sollten vorzugsweise eine Länge von 5 bis 200 cm, besonders bevorzugt von 20 bis 100 cm aufweisen. Sofern die Lagen nicht die benötigte Länge und Breite aufweisen, können sie zugeschnitten werden. Auch die Schläuche können, sofern sie nicht die benötigte Länge aufweisen, zugeschnitten werden. Aus diesem Grund kann das Kit eine Einrichtung zum Zuschneiden des Schaumstoffkörpers und des Schlauches umfassen.

Die Lagen, insbesondere die äußeren Oberflächen der beiden äußeren Lagen können beispielsweise mit Silikon oder einem anderen Anti-Haftmittel behandelt oder bedampft werden, um ein Verkleben der Lagen mit dem Organ, das die Körperhöhle bildet, zu verhindern.

Das Kit kann ferner eine Saugpumpe, insbesondere eine Handpumpe oder eine elektrische Saugpumpe, zum Erzeugen eines Unterdruckes in der Körperöffnung umfassen, wobei die Handpumpe zum Anschluß an das Ende eines Schlauches eingerichtet ist, das sich nicht zwischen den beiden Lagen befindet. Das Kit kann weiterhin Kopplungselemente zur Verbindung eines Schlauches mit einem anderen Schlauch oder Verlängerungselementen; Verlängerungselemente zur Verlängerung der Schläuche; Verteilerelemente zum Verteilen der Saugwirkung der Pumpe auf mehrere Schläuche; sowie ein Anschlußelement zum Anschließen eines oder mehrerer Schläuche an die Pumpe umfassen.

Die Lagen und der Schlauch können nach medizinischen Notwendigkeiten, die das Entfernen von Wundsekreten aus einer Körperhöhle erfordern, hergerichtet werden. Beispielsweise können zwei Lagen des Schaumstoffmaterials in die Körperhöhle eines Patienten eingebracht und ein Teil eines Schlauches zwischen die beiden Körperöffnungen eingebracht werden. Der Schlauch wird so verlegt, daß er aus der Körperhöhle und aus dem Körper eines Patienten hinausgeführt wird. Die Körperhöhle wird dann, sofern erforderlich operativ verschlossen. Anschließend wird das offene Ende des Schlauches, das sich außerhalb des Körpers befindet, an eine Saugpumpe angeschlossen.

Alternativ kann das Zuschneiden von zumindest zwei Lagen des Schaumstoffkörpers vorgesehen sein, so daß deren Abmessungen der Körperöffnung entsprechen, in die die Lagen eingebracht werden sollen. Anschließend wird zumindest ein Schlauch auf eine solche Länge zugeschnitten, daß ein Teil des Schlauches mit einem offenen Ende zwischen die beiden Lagen eingebracht werden kann und das andere offene Ende des Schlauches aus der Körperöffnung aus dem Körper eines Patienten hinausgeführt werden kann. Die Lagen und ein Teil des Schlauches werden dann - wie beschrieben - in die Körperhöhle eingebracht. Die Körperhöhle wird dann, sofern erforderlich, operativ verschlossen und das offene Ende des Schlauches, das sich außerhalb des Körpers des Patienten befindet, an die Saugpumpe angeschlossen.

Ein Verfahren zur Herstellung der erfindungsgemäßen Vorrichtung umfaßt somit
(a) das Zuschneiden von zumindest zwei Lagen des Schaumstoffkörpers, so daß deren Abmessungen der Körperöffnung entsprechen, in die die Lagen eingebracht werden sollen; und
(b) das Zuschneiden zumindest eines Schlauches auf eine solche Länge, daß ein Teil des Schlauches mit einem offenen Ende zwischen die beiden Lagen eingebracht werden kann und das andere offene Ende des Schlauches aus der Körperöffnung aus dem Körper eines Patienten hinausgeführt werden kann.

Ferner kann dieses Verfahren das Anschließen des offenen Enden der Schläuche, die sich außerhalb des Körpers des Patienten befinden, an eine Pumpe umfassen.

Das abgesaugte Wundsekret kann zur Überwachung des Zustandes der Körperhöhle verwendet werden. Beispielsweise können qualitative und quantitative Messungen in dem Wundsekret durchgeführt werden.

Die erfindungsgemäße Vorrichtung fördert eine permanente und vollständige Entfernung von Wundsekreten aus Körperhöhlen. Die Durchblutung an den Saugstellen wird: verbessert, Ödeme werden verkleinert, die Gefahr eines Kompartmentsyndroms wird verringert und die Säuberung und Heilung werden angeregt. Die Anzahl operativer Eingriffe kann deutlich verringert werden. Überdies wird eine Heilung einer in der Körperhöhle befindlichen Wunde durch eine verbesserte Durchblutung gefördert. Aufgrund der Flexibilität und Weichheit des Polyurethan-Schaumstoffkörpers kann dieser ohne Komplikationen in die Körperhöhle eingebracht werden. Aufgrund des andauernden Verschlusses der Körperhöhle unter Verwendung der erfindungsgemäßen Vorrichtung kann das Risiko einer externen Keimbesiedlung durch tägliche Operationen verringerte werden.

Aufgrund des erfindungsgemäß vorgesehenen Schaumstoffkörpers besitzt die erfindungsgemäße Vorrichtung eine um ein Vielfaches größere Saugoberfläche als bisher bekannte Vorrichtungen. Es kann die gesamte Körperhöhle, beispielsweise die gesamte Bauchhöhle, die eine im Vergleich zu anderen Körperhöhlen riesige Oberfläche aufweist, unter Verwendung der erfindungsgemäßen Vorrichtung besaugt werden. Der Schaumstoffkörper kann über mehrere Tage in der Körperhöhle verbleiben. Somit kann der Patient zwischenzeitlich ohne Narkose auskommen.

Der Schaumstoffkörper der Vorrichtung ist ein flexibles, organschonendes und sterilisierbares Material. Dieses Material in Form einer oder mehrerer Lagen und die notwendige Zahl an Schläuchen werden unter Narkose in eine Körperhöhle eines Patienten eingebracht. Eine Mobilisierung des Patienten nach dieser Operation ist ohne weiteres möglich; ebenso eine orale Kostaufnahme durch den Patienten. Die Zahl an Operationen und damit die Zahl von Operationsnarben werden auf ein Minimum reduziert.

Die erfindungsgemäße Vorrichtung ermöglicht, daß kontinuierliche Entfernen von Toxinen, Bakterien, Stoffwechselprodukten, Wundflüssigkeit, Viren usw. aus Körperhöhlen. Alle Organzwischenräume können mit der Vorrichtung behandelt werden.

Die Vorrichtung ist nicht auf das Entfernen von Wundsekreten beschränkt. Ebenso können andere Körperflüssigkeiten aus Wundhöhlen entfernt werden.

Die erfindungsgemäße Vorrichtung kann auch zum Entfernen von Wundsekreten aus offenen Wunden verwendet werden.

Die Erfindung wird nachstehend anhand von Ausführungsformen unter Bezugnahme auf die beiliegenden Zeichnungen näher erläutert. Dabei zeigen
- Fig. 1: Querschnittsdarstellungen von Ausführungsformen der erfindungsgemäßen Vorrichtung;
- Fig. 2: eine Draufsicht auf eine Lage eines Schaumstoffkörpers, auf das drei Schläuche aufgebracht worden sind; und
- Fig. 3: Draufsichten auf Lagen, auf die S-förmige gekrümmt Schläuche aufgebracht sind.

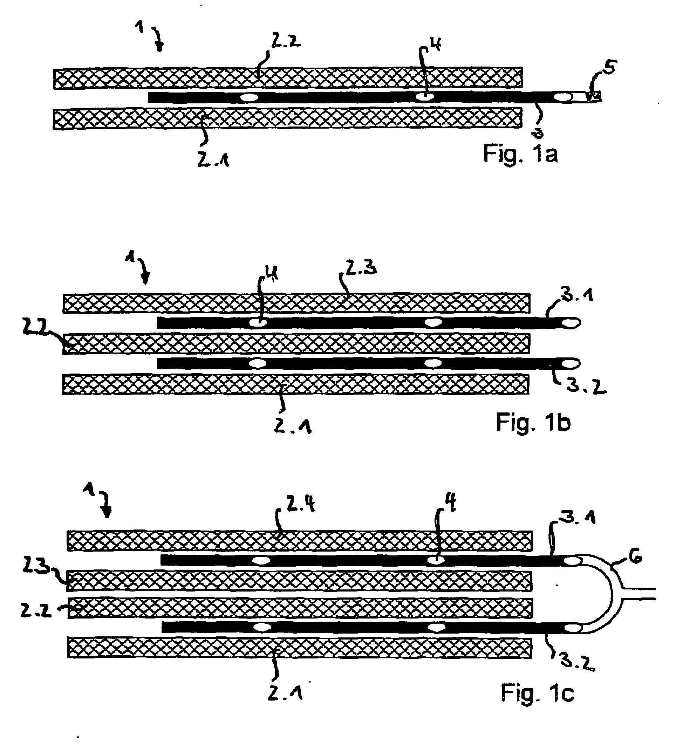

Fig. 1a ist eine schematische Querschnittsdarstellung einer Ausführungsform der erfindungsgemäßen Vorrichtung 1. Die Vorrichtung 1 besteht aus zwei Lagen 2.1 und 2.2 eines Schaumstoffmaterials (2), zwischen die ein Ende eines Schlauches 3 eingebracht ist. Der Abschnitt des Schlauches 3, der sich zwischen den beiden Lagen 2.1 und 2.2 befindet, weist zusätzliche Öffnungen 4 auf. Der andere Abschnitt des Schlauches 3, der sich nicht zwischen den beiden Lagen 2.1 und 2.2 befindet, kann aus der Körperhöhle herausgeführt werden und mittels eines Anschlußelementes 4 an einen anderen Schlauch oder ein Verlängerungselement angeschlossen werden.

Die in Fig. 1b gezeigte Ausführungsform gleicht der in Fig. 1a gezeigten Ausführungsform, außer daß eine weitere Lage 2.3 des Schaumstoffkörpers vorgesehen ist und ein weiterer Schlauch 3.2 zusätzlich zu dem ersten Schlauch 3.1 vorgesehen ist. Es ist jedoch nicht zwingend erforderlich, daß zwischen benachbarten Lagen in jedem Fall ein Schlauch eingebracht wird (siehe Fig. 1c). Die in Fig. 1c gezeigte Ausführungsform besteht aus vier Lagen 2.1, 2.2, 2.3 und 2.4 des Schaumstoffkörpers, wobei nur zwischen die Lagen 2.1 und 2.2 ein Schlauch 3.2 und zwischen die Lagen 2.3 und 2.4 ein Schlauch eingebracht wurde. Zwischen den Lagen 2.2 und 2.3 ist kein Schlauch vorgesehen, auch wenn dies benachbarte Lagen sind. Die Schläuche 3.1 und 3.2 sind mittels eines Verteilerelements 6 verbunden, das beispielsweise über ein Verlängerungselement an die Saugpumpe angeschlossen werden kann (nicht gezeigt).

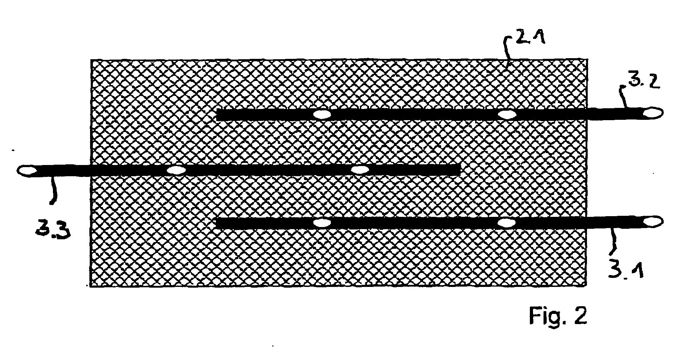

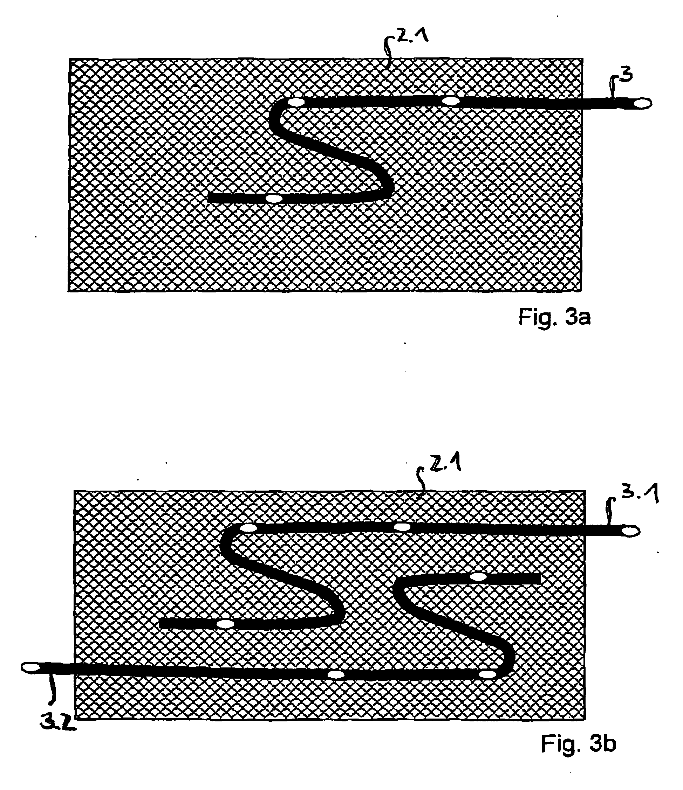

Fig. 2 zeigt eine Draufsicht auf eine Lage 2.1 des Schaumstoffkörpers, auf dem die Schläuche 3.1, 3.2 und 3.3 angeordnet sind. Auf den Schläuchen ist eine nicht gezeigte Lage 2.2 angeordnet. Es ist zu erkennen, daß nicht sämtliche Schläuche in derselben Richtung aus dem Schaumstoffkörper herausgeführt werden müssen. Die Figuren 3a und 3b zeigen ebenso wie Fig. 2 eine Lage 2.1 des Schaumstoffkörpers. In Fig. 3a ist der Abschnitt des Schlauches 3, der sich zwischen der Lage 3.1 und der darauf befindlichen Lage 3.2 (nicht gezeigt) befindet, S-förmig auf der Lage 2.1 angeordnet. In Fig. 3b ist zusätzlich ein zweiter Schlauch 3.2 vorgesehen, der ebenfalls einen S-förmigen Abschnitt aufweist.

### Bezugszeichenliste

- 1: Vorrichtung
- 2: Schaumstoffkörper
- 2.1-2.4: Lagen des Schaumstoffkörpers
- 3: Schlauch
- 4: weitere Öffnungen des Schlauches 3
- 5: Anschlußelement
- 6: Verteilerelement

## Patentansprüche

1. Vorrichtung zum Entfernen von Sekreten aus Körperhöhlen, umfassend
(a) zumindest zwei aufeinanderliegende Lagen eines Schaumstoffkörpers aus offenporigem Polyurethan, wobei das Polyurethan eine Porengröße von 10 bis 75 ppi und eine Rohdichte von 14 bis 75 kg/m³ aufweist, und
(b) zumindest einen Schlauch mit offenen Enden, dessen eines offene Ende zwischen zwei benachbarte Lagen des Schaumstoffkörpers eingebracht ist, wobei sich das zweite offene Ende des Schlauches außerhalb der Lagen befindet,
**dadurch gekennzeichnet, daß** die Lagen keine. Beschichtung aufweisen und daß nach dem Einbringen der Vorrichtung in die Körperhöhle, die Außenseiten der äußeren Lagen an den Innenseiten der Körperhöhle anliegen, so daß das Sekret über die Poren der beiden äußeren Lagen in den SchaumstoffkÖrper eintreten kann.

2. Vorrichtung nach Anspruch 1, **dadurch gekennzeichnet, daß** eine Lage des Schaumstoffkörpers eine Stärke von 3 bis 15 mm aufweist.

3. Vorrichtung nach einem der vorstehenden Ansprüche, **dadurch gekennzeichnet, daß** der Teil des Schlauches, der sich zwischen den beiden Lagen des Schaumstoffkörpers befindet, neben dem offenen Ende weitere Öffnungen aufweist, die im Mantel des Schlauches ausgebildet sind.

4. Vorrichtung nach Anspruch 3, **dadurch gekennzeichnet, daß** die weiteren Öffnungen gleichmäßig über den Teil des Schlauches, der sich zwischen den beiden Lagen befindet, verteilt sind.

5. Vorrichtung nach Anspruch 3, **dadurch gekennzeichnet, daß** die Größe jeder Öffnung der Größe des offenen Endes des Teiles des Schlauches, der sich zwischen den beiden Lagen befindet, gleicht.

6. Vorrichtung nach einem der Ansprüche 3 bis 5, **dadurch gekennzeichnet, daß** der Abstand zwischen zwei benachbarten Öffnungen sowie der Öffnung, die dem offenen Ende des Teiles des Schlauches, der sich zwischen den beiden Lagen befindet, benachbart ist, zwischen dem Zweifachen und dem Zehnfachen des Durchmessers einer Öffnung liegt.

7. Vorrichtung nach einem der Ansprüche 3 bis 6, **dadurch gekennzeichnet, daß** die Öffnungen auf dem Schlauchmantel versetzt zueinander angeordnet sind.

8. Vorrichtung nach einem der vorstehenden Ansprüche, **dadurch gekennzeichnet, daß** der Teil des Schlauches, der sich zwischen den beiden Lagen befindet, S-förmig gekrümmt zwischen den beiden Lagen angeordnet ist.

9. Vorrichtung nach einem der vorstehenden Ansprüche, **dadurch gekennzeichnet, daß** zwischen die beiden Lagen ein zweiter Schlauch mit offenen Enden eingebracht ist, dessen eines offene Ende zwischen die beiden Lagen des Schaumstoffkörpers eingebracht ist, wobei sich das zweite offene Ende des zweiten Schlauches außerhalb der Lagen befindet.

10. Vorrichtung nach einem der vorstehenden Ansprüche, **dadurch gekennzeichnet, daß** das Ende eines Schlauches, das sich außerhalb der Lagen befindet, mit einer Einrichtung zur Erzeugung von Unterdruck verbunden ist.

11. Kit zur Herstellung einer Vorrichtung nach einem der Ansprüche 1 bis 10, umfassend
(a) eine oder mehrere Lagen eines Schaumstoffkörpers aus offenporigem Polyurethan, wobei das Polyurethan eine Porengröße von 10 bis 75 ppi und eine Rohdichte von 14 bis 75 kg/m³ aufweist, und
(b) einen oder mehrere Schläuche mit offenen Enden, dessen eines offene Ende zwischen die beiden Lagen des Schaumstoffkörpers eingebracht wird, wobei sich das zweite offene Ende des Schlauches außerhalb der Lagen befindet;
**dadurch gekennzeichnet, daß** die Lagen keine Beschichtung aufweisen und daß nach dem Einbringen, der Vorrichtung in die Körperhöhle die Außenseiten der äußeren Lagen an den Innenseiten der Körperhöhle anliegen so daß das Sekret über die Poren der beiden äußeren Lagen in den Schaumstoffkörper eintreten kann.

12. Kit nach Anspruch 11, **dadurch gekennzeichnet, daß** es ferner eine Handpumpe zum Erzeugen eines Unterdruckes in der Körperöffnung umfaßt, wobei die Pumpe zum Anschluß an das Ende eines Schlauches eingerichtet ist, das sich nicht zwischen den beiden Lagen befindet.

## Claims

1. A device for removing secretions from body cavities, comprising
(a) at least two overlying sheets of a foam body made of an open porous polyurethane, said polyurethane having a pore size of from 10 to 75 ppi and a crude density of from 14 to 75 kg/m³, and
(b) at least one tube having open ends, one of which open ends is interposed between two adjacent sheets of the foam body and the second open end of the tube is located outside the sheets.
**characterized in that** the sheets have no coating and that after introducing the device into the body cavity the outsides of the outer sheets are adjacent to the insides of the body cavity, so that the secretion can enter the foam body through the pores of the two outer sheets.

2. The device according to claim 1, **characterized in that** one sheet of the foam body has a thickness of from 3 to 15 mm.

3. The device according to any one of the preceding claims, **characterized in that** the portion of the tube located between the two sheets of the foam body has further openings in addition to the open end formed in the sheath of the tube.

4. The device according to claim 3, **characterized in that** said further openings are distributed evenly across the portion of the tube located between the two sheets.

5. The device according to claim 3, **characterized in that** the size of each opening is equal to the size of the open end of the portion of the tube located between the two sheets.

6. The device according to any one of claims 3 to 5, **characterized in that** the distance between two adjacent openings as well as of the opening adjacent to the open end of the portion of the tube located between the two sheets is between the twofold and the tenfold of the diameter of one opening.

7. The device according to any one of claims 3 to 6, **characterized in that** the openings on the sheath of the tube are staggered.

8. The device according to any one of the preceding claims, **characterized in that** the portion of the tube located between the two sheets is arranged S-shaped curved between the two sheets.

9. The device according to any one of the preceding claims, **characterized in that** there is a second tube having open ends interposed between the two sheets, one of which open ends is interposed between the two sheets of the foam body, wherein the second open end of the second tube is located outside the sheets.

10. The device according to any one of the preceding claims, **characterized in that** the end of one tube located outside the sheets is connected with a device for generating under pressure.

11. A kit for preparing a device according to any one of claims 1 to 10, comprising
(a) one or more sheets of a foam body made of an open porous polyurethane, said polyurethane having a pore size of from 10 to 75 ppi und a crude density of from 14 to 75 kg/m³, and
(b) at least one tube having open ends, one of which open ends will be interposed between two adjacent sheets of the foam body, and the second open end of the tube is located outside the sheets,
**characterized in that** the sheets have no coating and that after introducing the device into the body cavity the outsides of the outer sheets are adjacent to the insides of the body cavity, so that the secretion can enter the foam body through the pores of the two outer sheets.

12. The kit according to claim 11, **characterized in that** it further comprises a hand pump for generating under pressure in the body orifice, wherein the pump is adapted for attachment to the end of a tube not located between the two sheets.

## Revendications

1. Dispositif de retrait de sécrétion hors des cavités corporelles, comprenant
(a) au moins deux couches superposées d'un corps en mousse synthétique en polyuréthane à pores ouverts, le polyuréthane présentant une grosseur de pore de 10 à 75 ppi et une densité brute de 14 à 75 kg/m³ et
(b) au moins un tuyau à extrémités ouvertes dont une extrémité ouverte est logée entre deux couches voisines du corps en mousse synthétique, la deuxième extrémité ouverte du tuyau se trouvant hors des couches,
**caractérisé en ce que** les couches ne présentent aucun revêtement et qu'après l'introduction du dispositif dans la cavité corporelle, les faces extérieures des couches extérieures posent contre les faces intérieures de la cavité corporelle de façon que la sécrétion puisse pénétrer dans le corps en mousse synthétique par l'intermédiaire des pores des deux couches extérieures.

2. Dispositif selon la revendication 1 **caractérisé en ce qu'**une couche du corps en mousse synthétique présente une épaisseur de 3 à 15 mm.

3. Dispositif selon l'une des revendications précédentes **caractérisé en ce que** la partie du tuyau se trouvant entre les deux couches du corps en mousse synthétique présente à côté de l'extrémité ouverte d'autres ouvertures formées dans la gaine du tuyau.

4. Dispositif selon la revendication 3 **caractérisé en ce que** les autres ouvertures sont réparties de façon régulière sur la partie du tuyau qui se trouve entre les deux couches.

5. Dispositif selon la revendication 3 **caractérisé en ce que** la dimension de chaque ouverture est semblable à la dimension de l'extrémité ouverte de la partie du tuyau qui se trouve entre les deux couches.

6. Dispositif selon l'une des revendications 3 à 5 **caractérisé en ce que** la distance entre deux ouvertures voisines ainsi que par rapport à l'ouverture voisine de l'extrémité ouverte de la partie du tuyau se trouvant entre les deux couches se situe entre le double et le décuple du diamètre d'une ouverture.

7. Dispositif selon l'une des revendications 3 à 6 **caractérisé en ce que** les ouvertures sont disposées sur la gaine du tuyau décalées les unes par rapport aux autres.

8. Dispositif selon l'une des revendications précédentes **caractérisé en ce que** la partie du tuyau se trouvant entre les deux couches est disposée courbée en forme de S entre les deux couches.

9. Dispositif selon l'une des revendications précédentes **caractérisé en ce qu'**entre les deux couches, un deuxième tuyau à extrémités ouvertes est logé dont une extrémité ouverte est logée entre les deux couches du corps en mousse synthétique, la deuxième extrémité ouverte du deuxième tuyau se trouvant hors des couches.

10. Dispositif selon l'une des revendications précédentes **caractérisé en ce que** l'extrémité d'un tuyau se trouvant hors des couches est associée à un dispositif de génération de sous-pression.

11. Kit de fabrication d'un dispositif selon l'une des revendications 1 à 10 comprenant:
(a) une ou plusieurs couches superposées d'un corps en mousse synthétique en polyuréthane à pores ouverts, le polyuréthane présentant une grosseur de pore de 10 à 75 ppi et une densité brute de 14 à 75 kg/m³ et
(b) un ou plusieurs tuyaux à extrémités ouvertes dont une extrémité ouverte est logée entre les deux couches du corps en mousse synthétique, la deuxième extrémité ouverte du tuyau se trouvant hors des couches;
**caractérisé en ce que** les couches ne présentent aucun revêtement et qu'après l'introduction du dispositif dans la cavité corporelle, les faces extérieures des couches extérieures posent contre les faces intérieures de la cavité corporelle de façon que la sécrétion puisse pénétrer dans le corps en mousse synthétique par l'intermédiaire des pores des deux couches extérieures.

12. Kit selon la revendication 11 **caractérisé en ce qu'**il contient de plus une pompe manuelle pour générer une sous-pression dans l'ouverture du corps, la pompe étant organisée pour être raccordée à l'extrémité d'un tuyau qui ne se trouve pas entre les deux couches.
